# EUROPÄISCHE PATENTSCHRIFT

(11) **EP 2 948 555 B1**
(45) Veröffentlichungstag und Bekanntmachung des Hinweises auf die Patenterteilung: **03.01.2018**
(21) Anmeldenummer: 14700294.3
(22) Anmeldetag: 10.01.2014
(51) Int. Cl.: C12P 7/02, C12P 7/16, C12P 7/18, C07C 29/136, C07C 67/08, C12P 7/62

(54) **VERFAHREN ZUR HERSTELLUNG VON ALPHA, OMEGA-ALKANDIOL**
PROCESS FOR PREPARING ALPHA, OMEGA ALKANEDIOLS
PROCÉDÉ DE FABRICATION D'ALPHA, OMÉGA-ALCANE-DIOL

(30) Priorität: 24.01.2013 EP 13152491
(43) Veröffentlichungstag der Anmeldung: 02.12.2015
(73) Patentinhaber: Evonik Degussa GmbH, 45128 Essen (DE)
(72) Erfinder: HAAS, Thomas, 48161 Münster (DE); ENGEL, Philip, 45147 Essen (DE); PFEFFER, Jan Christoph, 63452 Hanau (DE); THUM, Oliver, 40880 Ratingen (DE); GEHRING, Christian, 45770 Marl (DE)
(86) Internationale Anmeldenummer: PCT/EP2014/050373
(87) Internationale Veröffentlichungsnummer: WO 2014/114505

(56) Entgegenhaltungen:
- EP-A1- 2 602 329
- EP-A2- 2 322 598
- WO-A1-97/31882
- WO-A1-2009/077461
- WO-A1-2011/131420
- WO-A1-2013/083412
- DE-A1- 19 720 657

## Beschreibung

Die Erfindung betrifft ein Verfahren zur Herstellung von α, ω-Alkandiol umfassend die Schritte a) Umsetzen einer Alkansäure mit einem Alkanol zu einem Ester, b) Oxidation wenigstens eines terminalen Kohlenstoffatoms des Esters durch Kontaktieren mit einem Ganzzellkatalysator, der eine Alkanhydroxylase exprimiert, in wässriger Lösung und in Gegenwart von molekularem Sauerstoff, zu einem oxidierten Ester, c) Hydrieren des oxidierten Esters unter Entstehung eines α,ω-Alkandiols und eines Alkanols, und d) destillatives Abtrennen des Alkanols unter Enstehung einer mit Hinblick auf das Alkanol abgereicherten Reaktionsmischung, und Rückführen des Alkanols in Schritt a). DE19720657 offenbart ein Verfahren zur Herstellung von aliphatischen Alkoholen durch Hydrierung von aliphatischen Carbonsäuren, Anhydriden oder Estern unter Produktrückführung bei kontinuierlicher Fahrweise. Beispielhaft wird die Hydrierung von Adipinsäure (Dicarbonsäure) genannt. Ein Verfahrensschritt zur Herstellung der Adipinsäureester ist nicht beschrieben.

Amine und Diamine sind eine Klasse industriell stark nachgefragter Moleküle, die herkömmlich beim Cracken von Kohlenwasserstoffen gewonnen werden. Sie werden beispielsweise zur Herstellung von Polyamiden, synthetischen, im Handel erhältlichen, thermoplastischen Kunststoffen, eingesetzt.

Die konventionelle chemisch-technische Erzeugung von Aminen und Diaminen ist von der Versorgung mit fossilen Rohstoffen abhängig, ineffizient, und dabei fallen große Mengen unerwünschter Nebenprodukte an. Angesichts dieser Nachteile wurden Verfahren entwickelt, um Amine unter Verwendung von Biokatalysatoren ausgehend von nachwachsenden Rohstoffen zu gewinnen. Als Edukte kommen insbesondere Alkane oder Alkanole in Frage, die über biotechnologische Verfahren aus nachwachsenden Rohstoffen hergestellt werden können. Verschiedene Verfahren zur Umwandlung von Alkanen und Alkanolen zu Aminen sind im Stand der Technik beschrieben. Ein Nachteil der im Stand der Technik beschriebenen Verfahren besteht darin, dass nur ein Teil des als Edukt eingesetzten Alkans oder Alkanols zum Produkt umgewandelt wird. Ein erheblicher Anteil reagiert zu unerwünschten Neben- oder Zwischenprodukten oder verbleibt ohne abzureagieren in der Reaktionsmischung.

Vor diesem Hintergrund besteht das der Erfindung zu Grunde liegende Problem, ein Verfahren zur Herstellung eines α, ω-Alkandiols bereitzustellen, bei dem ein möglichst hoher Anteil des als Edukt eingesetzten Alkanols in das erwünschte Produkt, bevorzugt das interessierende α, ω-Alkandiol, eingebaut wird.

Diese und weitere Aufgaben werden durch den Gegenstand der vorliegenden Anmeldung und insbesondere auch durch den Gegenstand der beigefügten unabhängigen Ansprüche gelöst, wobei sich Ausführungsformen aus den Unteransprüchen ergeben.

Das der Erfindung zu Grunde liegende Problem wird gelöst durch ein Verfahren umfassend die Schritte
a) Umsetzen einer Alkansäure mit einem Alkanol zu einem Ester,
b) Oxidation wenigstens eines terminalen Kohlenstoffatoms des Esters durch Kontaktieren mit einem Ganzzellkatalysator, der eine Alkanhydroxylase exprimiert, in wässriger Lösung und in Gegenwart von molekularem Sauerstoff, zu einem oxidierten Ester,
c) Hydrieren des oxidierten Esters unter Entstehung eines α, ω-Alkandiols und eines Alkanols,
d) Destillatives Abtrennen des Alkanols unter Entstehung einer mit Hinblick auf das Alkanol abgereicherten Reaktionsmischung, und
e) Rückführen des Alkanols in Schritt a).
Bei der Alkansäure in Schritt a) kann es sich um eine Alkansäure der Formel H₃C - (CH₂)ₙ - COOH handeln und wobei n > 0, bevorzugt 2 bis 16 ist. Bei der Alkansäure kann es sich um Buttersäure handeln. Bei der Alkansäure in Schritt a) kann es sich um ein carboxyliertes Cycloalkan der Formel CₙH₂ₙ₋₁COOH handeln, wobei n > 4, bevorzugt 5 bis 8 ist. Das im Verfahren eingesetzte Alkanol kann ein Alkanol der Formel CₙOH₂ₙ₊₂ sein, und wobei n > 0. Die Alkansäure und das Alkanol in Schritt a) können das gleiche Kohlenstoffgerüst aufweisen. Der Ganzzellkatalysator in Schritt a), der eine Alkanhydroxylase exprimiert, kann ausgewählt werden aus der Gruppe umfassend AlkB aus *Pseudomonas putida* und Varianten davon und CYP153 aus *Alcanivorans borkumensis* SK2 und Varianten davon, wobei eine Variante der jeweiligen vorgenannten Aminosäuresequenz eine Sequenz umfasst, welche im Hinblick auf die entsprechende ursprüngliche Wildtypaminosäuresequenz eine Identität vom 70% oder mehr Prozent aufweist. Schritt a) kann mittels säurekatalysierter Esterbildung durchgeführt werden, bevorzugt in Anwesenheit eines organischen Lösungsmittels.

Schritt a) kann mittels Kontaktieren mit einer Esterase, Protease oder Lipase, bevorzugt bereitgestellt in Form eines Ganzzellkatalysators exprimierend die Esterase oder Lipase, in wässriger Lösung durchgeführt werden. Die Alkansäure in Schritt a) kann durch Oxidation eines Alkans zur Alkansäure bereitgestellt werden. Das erfindungsgemäße Verfahren sieht zunächst in Schritt a) das Umsetzen einer Alkansäure mit einem Alkanol zu einem Ester vor. Dem Fachmann sind dafür geeignete Reaktionsbedingungen bekannt. Beispielsweise kann die Veresterung direkt unter Säurekatalyse durchgeführt werden. Der Ester wird in diesem Fall bevorzugt aus dem Gleichgewicht mit Säure und Alkohol destillativ abgezogen. Bei dem Alkanol handelt es sich bevorzugt um ein Alkanol der Formel CₙOH₂ₙ₊₂, wobei n größer als 0 und bevorzugt 1 bis 4, am bevorzugtesten 4 und beispielsweise 1, 2, 3, 4, 5, 6, 7, 8, 9, 10, 11, 12, 13, 14, 15, 16, 17, 18, 19, 20, 21 oder 22 ist.

Schritt a) wird bevorzugt in Anwesenheit eines organischen Lösungsmittels ausgeführt. Als Lösungsmittel eigenen sich insbesondere sekundäre und tertiäre Alkohle, insbesondere verzweigte Alkohole wie 2-Methyl-2-Butanol. Weiterhin geeignet sind Ether wie tert-ButylMethyl-Ether oder Cycloalkane wie Hexan, Methylhexan oder aromatische Lösungsmittel wie Toluol.

In einer bevorzugten Ausführungsform wird Schritt a) durch Kontaktieren mit einer Hydrolase, bevorzugt aus der Gruppe umfassend Esterase, Protease oder Lipase, durchgeführt. Geeignete Hydrolasen sind im Stand der Technik beschrieben. Besonders geeignet ist Beispielsweise die Lipase Novozym® 435 der Firma Novozymes.

Nach Schritt a) erfolgt in Schritt b) die Oxidation wenigstens eines terminalen Kohlenstoffatoms des Esters durch Kontaktieren mit einem Ganzzellkatalysator, der eine Alkanhydroxylase exprimiert, in wässriger Lösung und in Gegenwart von molekularem Sauerstoff, zu einem oxidierten Ester. Unter dem Begriff "Ganzzellkatalysator", wie hierin verwendet, versteht man eine intakte, lebensfähige und metabolisch aktive Zelle, die die erwünschte enzymatische Aktivität der Alkanhydroxylase, bereitstellt. Bevorzugt handelt es sich bei dem Ganzzellkatalysator um eine prokaryotische, bevorzugt um eine bakterielle Zelle. In einer weiteren bevorzugten Ausführungsform handelt es sich um eine Säugetierzelle. In einer weiteren bevorzugten Ausführungsform handelt es sich um eine niedere eukaryotische Zelle, bevorzugt um eine Hefezelle. Beispielhafte prokaryotische Zellen umfassen *Escherichia,* besonders *Escherichia coli,* und Stämme der Gattung *Pseudomonas* und *Corynebacterium.* Beispielhafte niedere eukaryotische Zellen umfassen die Gattungen *Saccharomyces, Candida, Pichia, Yarrowia, Schizosaccharomyces,* besonders die Stämme *Candida tropicalis, Schizosaccharomyces pombe, Pichia pastoris, Yarrowia lipolytica* und *Saccharomyces cerivisiae.* Dem Fachmann sind geeignete Ganzzellkatalysatoren, Wege zu deren Herstellung durch gentechnische Arbeiten und Vorgehensweisen zum Kultivieren bekannt.

Für die Oxidation in Schritt b) ist es essentiell, dass der Ganzzellkatalysator eine Alkanhydroxylase aufweist. Weiterhin ist es vorteilhaft, wenn der erfindungsgemäße Ganzzellkatalysator weitere, für die Aktivität der Alkanhydroxylase förderliche Enzyme aufweist. In einer bevorzugten Ausführungsform wird unter dem Begriff "Alkanhydroxylase", wie hierin verwendet, ein Enzym verstanden, das die Hydroxylierung von unsubstituierten linearen Alkylresten umfassend wenigstens sechs, bevorzugt zwölf Kohlenstoffstoffreste katalysiert. Beispielhafte Oxidationssysteme sind u. a. in der PCT/EP 2008/067447 (WO 2009/077461) beschrieben.

Bevorzugt handelt es sich bei der Alkanhydroxylase um eine Cytochrom P450-Monooxygenase der CYP153-Familie. In einer bevorzugten Ausführungsform wird unter dem Begriff "Cytochrom P450-Monooxygenase der CYP153-Familie" eine cytosolische Oxidase verstanden, die Teil eines 3 Komponenten-Systems ist, das weiterhin ein Ferredoxin und eine Ferredoxin-Reduktase umfasst, mit einer Alkan-Bindestelle und der Fähigkeit, Alkane zu hydroxylieren. In einer besonders bevorzugten Ausführungsform handelt es sich um ein Enzym, das zu wenigstens 80, bevorzugt 90, am bevorzugtesten 95 oder 99 Prozent Sequenzidentität zur Cytochrom P450-Monooxygenase der CYP153-Familie aus *Alcanivorax borkumensis* SK2 (Datenbankcode YP_691921) aufweist oder um ein Enzym, das eine Polypeptidsequenz umfasst, die wenigstens 80, bevorzugt 90, am bevorzugtesten 95 oder 99 Prozent Sequenzidentität zur Cytochrom P450-Monooxygenase der CYP153-Familie aus *Alcanivorax borkumensis* SK2 (Datenbankcode YP_691921) aufweist und darüber hinaus Alkanhydroxylase-Aktivität aufweist. Die genannten Datenbankcodes beziehen sich dabei, wie durchgehend in dieser Anmeldung, auf die NCBI (National Center for Biotechnology Information, Bethesda, USA)-Datenbanken, genauer gesagt die am 11. Januar 2013 online verfügbare Version. In einer bevorzugten Ausführungsform wird unter dem Begriff "Cytochrom P450-Monooxygenase der CYP153-Familie" eine nicht membrangebundene Oxidase verstanden, die eine Bindestelle für Alkane, unsubstituierte lineare Alkylreste umfassend wenigstens fünf, bevorzugt zwölf Kohlenstoffstoffreste oder einfach hydroxylierte Alkane und deren Polypeptidkette das Motiv LL(I/L)(V/I)GGNDTTRN umfasst. In einer bevorzugten Ausführungsform handelt es sich bei einer "Cytochrom P450-Monooxygenase der CYP153-Familie", wie hierin verwendet, um eine Cytochrom P450-Monooxygenase der CYP153-Familie aus *Alcanivorax borkumensis* SK2 (Datenbankcode YP_691921) oder eine Variante davon, die bevorzugt Alkanhydroxylaseaktivität aufweist.

Zur optimalen Versorgung der Cytochrom P450-Monooxygenase der CYP153-Familie mit Elektronen aus dem Reduktionsmittel, bevorzugt NADH, ist es bevorzugt, dass der Ganzzellkatalysator die Alkanhydroxylase zusammen mit jeweils einer funktionell mit ihr wechselwirkenden Ferredoxin-Reduktase und einem funktionell mit ihr wechselwirkendem Ferredoxin exprimiert wird. Dabei handelt es sich um co-exprimierte Polypeptide oder um N- oder C-terminal mit der Cytochrom P450-Monooxygenase der CYP153-Familie fusionierte Polypeptide. Ob eine Ferredoxin-Reduktase oder ein Ferredoxin mit einer gegebenen Cytochrom P450-Monooxygenase der CYP153-Familie mit einander funktionell wechselwirken, kann der Fachmann leicht dadurch feststellen, ob das Reduktionsmittel in Gegenwart eines Alkansubstrates und der drei Polypeptide effizienter als für den Fall, dass wenigstens eines der drei fehlt, oxidiert wird. Alternativ kann der von Scheps, D., Malca, H., Hoffmann, B., Nestl, B. M, und Hauer, B. (2011) Org. Biomol. Chem., 9, 6727 beschriebene Enzymtest verwendet werden, der im Fall funktionell wechselwirkender Polypeptide eine deutliche Erhöhung der Reaktionsgeschwindigkeit zeigt. In einer besonders bevorzugten Ausführungsform stammen die Cytochrom P450-Monooxygenase der CYP153-Familie, das Ferredoxin und die Ferredoxin-Reduktase aus dem gleichen Organismus. In einer besonders bevorzugten Ausführungsform handelt es sich um die Ferredoxin-Reduktase aus *Alcanivorax borkumensis* SK2 (Datenbankcode YP_691923) oder eine Variante davon, das Ferredoxin aus *Alcanivorax borkumensis* SK2 (Datenbankcode YP_691920) oder eine Variante davon und die Cytochrom P450-Monooxygenase der CYP153-Familie aus *Alcanivorax borkumensis* SK2 (Datenbankcode YP_691921) oder eine Variante davon.

In einer weiteren bevorzugten Ausführungsform handelt es sich bei der Alkanhydroxylase um eine AlkB-Monooxygenase. AlkB stellt eine zunächst aus dem AlkBGT-System aus *Pseudomonas putida* Gpo1 bekannt gewordene Oxidoreduktase dar, die von zwei weiteren Polypeptiden, AlkG und AlkT, abhängig ist. AlkT wird als FAD-abhängige Rubredoxin-Reduktase charakterisiert, die Elektronen aus NADH an AlkG weitergibt. Bei AlkG handelt es sich um ein Rubredoxin, ein eisenhaltiges Redoxprotein, das als direkter Elektronendonor für AlkB fungiert. In einer bevorzugten Ausführungsform wird unter dem Begriff "AlkB-Monooxygenase" ein Polypeptid mit einer Sequenzhomologie von wenigstens, in der Reihenfolge zunehmender Bevorzugung angegeben, 75, 80, 85, 90, 92, 94, 96, 98 oder 99 % zur Sequenz des AlkB von *Pseudomonas putida* Gpo1 (Datenbankcode: CAB54050.1; dieser Datenbankcode stammt wie alle weiteren in der Anmeldung verwendeten aus dem Stand der Technik, nämlich aus der NCBI Datenbank, genauer dem am 11. Januar 2013 online verfügbaren Release) mit der Fähigkeit, Alkane zu oxidieren. In einer besonders bevorzugten Ausführungsform handelt es sich bei der AlkB-Monooxygenase um eine mit den AlkG (CAB54052.1)- und AlkT (CAB54063.1)-Polypeptiden aus *Pseudomonas putida* Gpo1 funktionell zusammenwirkende, Alkane oxidierende Oxidoreduktase. Zur optimalen Versorgung der AlkB-Alkanhydroxylase mit Elektronen ist es bevorzugt, dass die Zelle die Alkanhydroxylase zusammen mit funktionell mit ihr wechselwirkenden Hilfsproteinen, bevorzugt AlkG und/oder AlkT oder jeweils Varianten davon exprimiert wird, wobei es sich in einer besonders bevorzugten Ausführungsform wiederum um AlkG (CAB54052.1)- und AlkT (CAB54063.1)-Polypeptide aus *Pseudomonas putida* Gpo1 handelt.

Der in Schritt b) zur Oxidation verwendbare Ganzzellkatalysator kann auch verwendet werden, um die Alkansäure in Schritt b) bereitzustellen. Dies geschieht bevorzugt durch Kontaktieren des Ganzzellkatalysators mit einem Alkan der für die Alkansäure gewünschten Kettenlänge. Für diesen Fall muss der Ganzzellkatalysator eine Alkanhydroxylase exprimieren, die in der Lage ist, das terminale Kohlenstoffatom eines Alkans bis zur Carboxylgruppe zu oxidieren.

Die Lehre der vorliegenden Erfindung kann nicht nur unter Verwendung von Makromolekülen mit der exakten Aminosäure- oder Nukleinsäuresequenz, auf die hierin Bezug genommen wird, bzw. nicht nur unter Verwendung von einer Zelle mit relativ zum jeweiligen Wildtyp verringerter Aktivität eines Polypeptides mit der exakten Aminosäuresequenz, auf die hierin Bezug genommen wird, ausgeführt werden, sondern auch unter Verwendung einer Variante derartiger Makromoleküle oder von einer Zelle mit einer relativ zum jeweiligen Wildtyp der jeweiligen Zelle verringerten Aktivität einer Variante des Polypeptids, die durch Deletion, Addition oder Substitution einer oder mehr als einer Aminosäuren oder Nukleinsäuren erhalten werden kann. In einer bevorzugten Ausführungsform bedeutet der Begriff "Variante" einer Nukleinsäuresequenz oder Aminosäuresequenz, im Folgenden gleichbedeutend und austauschbar mit dem Begriff "Homologon" gebraucht, wie hierin verwendet, eine andere Nukleinsäure- oder Aminosäuresequenz, die eine Sequenz umfasst oder darstellt, welche mit Hinblick auf die entsprechende ursprüngliche Wildtyp-Nukleinsäure- oder -aminosäuresequenz eine Homologie, hier gleichbedeutend mit Identität verwendet, von 70, 75, 80, 85, 90, 92, 94, 96, 98, 99 % oder mehr Prozent aufweist, wobei bevorzugt andere als die das katalytisch aktive Zentrum ausbildende Aminosäuren oder für die Struktur, Aktivität oder Faltung essentielle Aminosäuren deletiert oder substituiert sind oder solche lediglich konservativ substituiert sind, beispielsweise ein Glutamat statt einem Aspartat oder ein Leucin statt einem Valin. Der Stand der Technik beschreibt Algorithmen, die verwendet werden können, um das Ausmaß von Homologie von zwei Sequenzen zu berechnen, z. B. Arthur Lesk (2008), Introduction to bioinformatics, 3rd edition. In einer weiteren bevorzugteren Ausführungsform der vorliegenden Erfindung weist die Variante einer Aminosäure- oder Nukleinsäuresequenz, bevorzugt zusätzlich zur oben genannten Sequenzhomologie, im Wesentlichen die gleiche enzymatische Aktivität des Wildtypmoleküls bzw. des ursprünglichen Moleküls auf. Zum Beispiel weist eine Variante eines als Protease enzymatisch aktiven Polypeptids die gleiche oder im Wesentlichen die gleiche proteolytische Aktivität wie das Polypeptidenzym auf, d.h. die Fähigkeit, die Hydrolyse einer Peptidbindung zu katalysieren. In einer besonderen Ausführungsform bedeutet der Begriff "im Wesentlichen die gleiche enzymatische Aktivität" eine Aktivität mit Hinblick auf die Substrate des Wildtyp-Polypeptids, die deutlich über der Hintergrundaktivität liegt oder/und sich um weniger als 3, bevorzugter 2, noch bevorzugter eine Größenordnung von den K_{M}- und/oder k_{cat}-Werten unterscheidet, die das Wildtyppolypeptid mit Hinblick auf die gleichen Substrate aufweist. In einer weiteren bevorzugten Ausführungsform umfasst der Begriff "Variante" einer Nukleinsäure- oder Aminosäuresequenz wenigstens einen aktiven Teil/oder Fragment der Nukleinsäure- bzw. Aminosäuresequenz. In einer weiteren bevorzugten Ausführungsform bedeutet der Begriff "aktiver Teil", wie hierin verwendet, eine Aminosäuresequenz oder eine Nukleinsäuresequenz, die eine geringere als die volle Länge der Aminosäuresequenz aufweist bzw. für eine geringere als die volle Länge der Aminosäuresequenz kodiert, wobei die Aminosäuresequenz oder die kodierte Aminosäuresequenz mit geringerer Länge als der Wildtyp-Aminosäuresequenz im Wesentlichen die gleiche enzymatische Aktivität wie das Wildtyppolypeptid oder eine Variante davon aufweist, beispielsweise als Protease. In einer besonderen Ausführungsform umfasst der Begriff "Variante" einer Nukleinsäure eine Nukleinsäure, deren komplementärer Strang, bevorzugt unter stringenten Bedingungen, an die Wildtyp-Nukleinsäure bindet. Die Stringenz der Hybridisierungsreaktion ist für den Fachmann leicht bestimmbar und hängt im Allgemeinen von der Länge der Sonde, den Temperaturen beim Waschen und der Salzkonzentration ab. Im Allgemeinen benötigen längere Sonden höhere Temperaturen zum Hybridisieren, wohingegen kürzere Proben mit geringen Temperaturen auskommen. Ob Hybridisierung stattfindet, hängt im Allgemeinen von der Fähigkeit der denaturierten DNA ab, an komplementäre Stränge zu annellieren, die in ihrer Umgebung vorhanden sind, und zwar unterhalb der Schmelztemperatur. Die Stringenz von Hybridisierungsreaktion und entsprechende Bedingungen sind ausführlicher in F M Ausubel (1995), Current Protocols in Molecular Biology. John Wiley & Sons, Inc. beschrieben. Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet in einer bevorzugten Ausführungsform unter stringenten Bedingungen statt, das heißt, es werden nur Hybride gebildet, bei denen Sonde und Zielsequenz, d. h. die mit der Sonde behandelten Polynukleotide, mindestens 70 % identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996). Für die Hybridisierungsreaktion kann beispielsweise ein Puffer entsprechend 5x SSC-Puffer bei einer Temperatur von ca. 50 °C - 68 °C eingesetzt werden. Dabei können Sonden auch mit Polynukleotiden hybridisieren, die weniger als 70 % Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von in der Reihenfolge zunehmender Bevorzugung ca. 50 °C - 68 °C, ca. 52 °C - 68 °C, ca. 54 °C - 68 °C, ca. 56 °C - 68 °C, ca. 58 °C - 68 °C, ca. 60 °C - 68 °C, ca. 62 °C - 68 °C, ca. 64 °C - 68 °C, ca. 66 °C - 68 °C eingestellt wird. Temperaturbereiche von ca. 64 °C - 68 °C oder ca. 66 °C - 68 °C werden bevorzugt. Es ist gegebenenfalls möglich die Salzkonzentration bis auf eine Konzentration entsprechend 0,2 x SSC oder 0,1 x SSC zu senken. Durch schrittweise Erhöhung der Hybridisierungstemperatur in Schritten von ca. 1 - 2 °C von 50 °C auf 68 °C können Polynukleotidfragmente isoliert werden, die beispielsweise in der Reihenfolge zunehmender Bevorzugung mindestens 70 % oder mindestens 80 % oder mindestens 90 %, mindestens 91 %, mindestens 92 %, mindestens 93 %, mindestens 94 %, mindestens 95 %, mindestens 96 %, mindestens 97 %, mindestens 98 % oder mindestens 99 % Identität zur Sequenz des eingesetzten Nukleinsäuremoleküls. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558). In einer bevorzugten Ausführungsform umfasst der Begriff "Variante" einer Nukleinsäure, wie hierin verwendet, eine beliebige Nukleinsäuresequenz, die für die gleiche Aminosäuresequenz wie die ursprüngliche Nukleinsäure oder eine Variante dieser Aminosäuresequenz im Rahmen der Degeneriertheit des genetischen Codes kodiert.

Bei der Verwendung eines Ganzzellkatalysators kann sich das Problem stellen, dass ein Substrat mit einem intrazellulär lokalisierten Enzym in Kontakt gebracht werden muss, damit es zur erwünschten Reaktion kommt. Im Falle langkettiger Alkane und Derivate davon ist bevorzugt, dass der Ganzzellkatalysator ein Polypeptid der AlkL-Familie aufweist. In einer bevorzugten Ausführungsform handelt es sich bei einem "Polypeptid der AlkL-Familie", wie hierin verwendet, um ein Polypeptid, das über eine Länge von 230 aufeinander abfolgenden Aminosäuren eine wenigstens 80, bevorzugt 90, noch bevorzugter 90%ige Sequenzidentität zu AlkL aus *Pseudomonas putida* (Datenbankcode CAB69081) und bevorzugt die Fähigkeit aufweist, den Import von langkettigen Alkanen ins Innere einer Zelle zu unterstützen. In einer weiteren Ausführungsform handelt es sich bei einem "Polypeptid der AlkL-Familie", wie hierin verwendet, um eine in der äußeren Membran eines Gram-negativen Bakteriums lokalisiertes Polypeptid, welches das Sequenzmotiv DXWAPAXQ(V/A)GXR, aufweist, wobei X eine proteinogene Aminosäure darstellt, und bevorzugt zusätzlich AlkL aus *Pseudomonas putida* (Datenbankcode CAB69081) oder eine Variante davon ist. Beispielhafte Mitglieder der AlkL-Familie umfassen AlkL aus *Pseudomonas putida* (Datenbankcode CAB69081), *Marinobacter aquaeolei* VT8 (Datenbankcode YP_957722), *Oceanicaulis alexandrii* HTCC2633 (Datenbankcode ZP_00953584), *Marinobacter manganoxydans* Mnl7-9 (Datenbankcode ZP_09158756), *Caulobacter* sp. K31 (Datenbankcode YP_001672217), *Pseudomonas oleovorans* (Datenbankcode Q00595) und Varianten davon.

Auch wenn die Erfindung mit unveränderten Wildtypstämmen von Ganzzellkatalysatoren, die eine Alkanhydroxylase aufweisen, ausgeführt werden kann, ist es bevorzugt dass es sich bei den erfindungsgemäß verwendeten Enzymen um rekombinant im Ganzzellkatalysator exprimierte Enzyme handelt. In einer bevorzugten Ausführungsform wird unter dem Begriff "rekombinant", wie hierin verwendet, verstanden, dass das kodierende Nukleinsäure-Molekül in der Natur nicht vorkommt und/oder es unter Verwendung von gentechnischen Methoden hergestellt wurde. In einer bevorzugten Ausführungsform spricht man von einem rekombinanten Protein, wenn das entsprechende Polypeptid von einer rekombinanten Nukleinsäure kodiert ist. In einer bevorzugten Ausführungsform wird unter einer rekombinanten Zelle, wie hierin verwendet, eine Zelle verstanden, die wenigstens eine rekombinante Nukleinsäure oder ein rekombinantes Polypeptid aufweist. Dem Fachmann sind zum Herstellen rekombinanter Moleküle oder Zellen geeignete Verfahren bekannt, beispielsweise die in Sambrook/Fritsch/Maniatis (1989): Molecular Cloning: A Laboratory Manual, Cold Spring Harbor Laboratory Press, 2nd edition, beschriebenen.

Als Substrat für die Alkanhydroxylase muss in Schritt b) molekularer Sauerstoff (O₂) anwesend sein. Dies wird bevorzugt durch Zugang von atmosphärischer Luft zur wässrigen Lösung erreicht, in der Schritt b) abläuft. Bevorzugt wird die wässrige Lösung unter Belüftung gerührt.

Als wässrige Lösung kommt eine jede Lösung auf Wasserbasis in Frage, die mit der Aktivität des Ganzzellkatalysators kompatibel ist. Bevorzugt handelt es sich um ein wässriges Medium, besonders bevorzugt um ein Minimalmedium, das den Ganzzellkatalysator stoffwechselaktiv erhält, aber neben den Edukten möglichst wenig weitere Substanzen umfasst, die die Gewinnung des Reaktionsproduktes in reiner Form erschweren würden. Beispielsweise kann M9-Medium verwendet werden.

Bei der Alkansäure kann es sich um eine beliebige organische Verbindung handeln, die eine veresterbare Carboxygruppe an einem Alkylrest trägt. Besonders bevorzugt handelt es sich um eine Alkansäure der Formel H₃C - (CH₂)ₙ - COOH, wobei n 0 oder größer ist, bevorzugt 2 bis 24, besonders bevorzugt 2 bis 16, am bevorzugtesten 4, beispielsweise 0, 1, 2, 3, 4, 5, 6, 7, 8, 9, 10, 11, 12, 13, 14, 15, 16, 17, 18, 19, 20, 21, 22, 23 oder 24. In einer weiteren bevorzugten Ausführungsform handelt es sich bei der Alkansäure um ein carboxyliertes Cylcoalkan der Formel CₙH₂ₙ₋₁COOH, wobei n 5, 6, 7, 8, 9, 10, 11, 12, 13, 14, 15, 16, 17, 18, 19, 20, 21, 22, 23 oder 24 und bevorzugt 5 bis 8 ist

Mit Hinblick auf die Alkansäure wie auf jede in dieser Anmeldung beschriebene chemische Verbindung gilt, dass die jeweilige angegebene Formel sämtliche Salze, protonierte oder deprotonierte der jeweiligen Verbindung umfasst. Beispielsweise umfasst die Buttersäure nicht nur die protonierte Form, sondern auch das Salz Butyrat mit sämtlichen Kationen, beispielsweise Natriumbutyrat.

Schritt c) des erfindungsgemäßen Verfahrens umfasst das Hydrieren des oxidierten Esters unter Entstehung des Alkandiols und Alkanols. Im Stand der Technik, beispielsweise in den Patentanmeldungen WO97/31882 und EP1042259B1, sind zahlreiche geeignete Hydrierungsverfahren beschrieben, beispielsweise unter Verwendung von ZnO/CuO-, Ru- oder Rh-haltigen Katalysatoren, woei mit anorganischen Hydriden wie LiAlH₄.

Schritt d) des erfindungsgemäßen Verfahrens umfasst das destillative Abtrennen des Alkanols unter Entstehung einer mit Hinblick auf das Alkanol abgereicherten Reaktionsmischung.

Letztere umfasst das Produkt Alkandiol, das im Folgenden durch weitere Trenn- und Waschschritte vom Medium und vom Ganzzellkatalysator abgetrennt werden kann.

Der letzte Schritte des Verfahrens umfasst das Rückführen des in Schritt d) abgetrennten Alkanols in Schritt a), d.h. das Alkanol wird mit weiterer Alkansäure umgesetzt und bleibt dem Verfahren somit erhalten.

Die vorliegende Erfindung wird weiterhin durch die folgenden Figuren und nicht beschränkenden Beispiele veranschaulicht, denen weitere Merkmale, Ausführungsformen, Aspekte und Vorteile der vorliegenden Erfindung entnommen werden können.
**Fig. 1** zeigt den zeitlichen Verlauf der Buttersäure- und 1-Butanol-Konzentration bei der Biotransformation von n-Butan mit *E.coli* W3110 pBT10, wie bei der Durchführung von Beispiel 1 entstanden.
**Fig. 2** zeigt die Massenspuren der HPLC-MS-Analyse der Oxidationsprodukte von Butylbutyrat bei Verwendung von *E.coli* W3110 pCOM10[Ab_Fd / CYP153-2 / FdOR / alkL] mit einem Vergleich der 10 min-Probe mit der 7h-Probe, wie sie bei Durchführung von Beispiel 2 entstanden.
**Fig. 3** zeigt die Massenspuren der HPLC-MS-Analyse der Oxidationsprodukte von Butylbutyrat bei Verwendung von *E.coli* W3110 pBT10 mit einem Vergleich der 10 min-Probe mit der 7 h-Probe, wie sie in Beispiel 2 entstanden.

### Beispiel 1: Oxidation von n-Butan durch E. coli W3110 pBT10 mit der Monooxygenase (alkBGT) aus P. putida GP01.

### a) Produktion von Biomasse im 10 I-Maßstab

Preseedkultur: 1 Liter LB Medium (5 g/l Hefeextrakt, 10 g/l Pepton, 0,5 g/l NaCl, in 1 L Wasser gelöst, autoklaviert für 20 Minuten bei 121 °C) wurde hergestellt.

Von dieser Lösung wurden je 5 x 25 ml in 100 ml-Schüttelkolben mit Schikanen gefüllt, mit je 25 µl einer steril filtrierten Kanamycin-Lösung (50 g/l) komplettiert und mit jeweils 200 µl einer Glycerin-Kryokultur von *E. coli* W3110 pBT10 angeimpft. Der Stamm enthaltend das Plasmid pBT10 wird bereits ausführlich in Beispiel 4 der WO13083412 beschrieben. Diese Kulturen wurden für 18 Stunden bei 37 °C und 200 rpm (Amplitude 2,5 cm) inkubiert.

Seedkultur: 1 Liter Hochzelldichtemedium (HZD-Medium) wurde hergestellt, bestehend aus 1,76 g/l NH₄SO₄, 19,08 g/l K₂HPO₄, 12,5 g/l KH₂PO₄, 6,66 g/l Hefeextrakt, 1,96 g/l Na₃-Citrat, 17 ml NH₄Fe-Citrat-Lösung (1 %), 5 ml Spurenelementelösung US3 (1 Liter der Spurenelementelösung US 3 setzt sich zusammen aus 36,5 g HCl 37 %, 1,91 g MnCl₂ x 4H₂O, 1,87 g ZnSO₄ x 7H₂O, 0,8 g Na-EDTA x 2H₂O, 0,3 g H₃BO₃, 0,25 g Na₂MoO₄ x 2H₂O, 4,7 g CaCl₂ x 2H₂O, 17,8 g FeSO₄ x 7 H₂O, 0,15 g CuCl₂ x 2H₂O, aufgelöst in 1 I Wasser.), 30 ml Feedlösung (Glucose 50 % w/v, MgSO₄ x 7 H₂O 0,5 % w/v, NH₄Cl 2,2 % w/v), 1 ml Kanamycin-Lösung (50 g/I). 948 ml Lösung mit NH₄SO₄ bis Na₃-Citrat wurden autoklaviert, der Rest wurde jeweils getrennt steril filtriert und im Anschluss steril zugegeben. Der pH lag bei 6,8.

5 x 75 ml des HZD- Mediums wurden in 1000 ml-Schüttelkolben mit Schikanen gegeben, mit jeweils 25 ml Preseedkultur inokuliert und für 30 h bei 37 °C und 200 rpm (Amplitude 2,5 cm) kultiviert.

Ein steriler 10 I-Fermenter wurde mit 7 I eines sterilen Mediums mit der Zusammensetzung 1,75 g/l (NH₄)₂SO₄, 19 g/l K₂HPO₄ x 3 H₂O, 12,5 g/l KH₂PO₄, 6,6 g/l Hefeextrakt, 2,24 g/l Na₃-Citrat x 2H₂O, 15 g/l Glucose, 0,49 g/l MgSO₄ x 7 H₂O, 16,6 ml/l NH₄Fe-Citrat-Lösung (1 % w/v), 15 ml/l Spurenelementlösung (wie in der Seedkultur), 1 ml/l Kanamycin-Lösung (50 mg/l) und 2 ml Antischaummittel Delamex befüllt. Als Feed wurde eine autoklavierte Lösung von Glucose (50 % w/v) mit MgSO₄ x 7H₂O 10g/l angeschlossen, zur pH Korrektur 0,5M H₂SO₄ und 25 % NH₄OH.

Die Kulturen aus den Schüttelkolben wurden steril vereinigt und über eine Transferflasche in den Fermenter inokuliert. Als Fermentationsbedingungen wurden eingestellt pO₂ 30 %, Airflow 6nlpm, Rührer 400 - 1200 rpm Temperatur 37 °C, pH 7, Feedstart 8 h, Feedrate 150 - 250 g/h. Nach 19 h wurde die Temperatur auf 30 °C gesenkt und mit 0,4 mM DCPK induziert. Nach 23 Stunden betrug die OD600 im Fermenter ca.100, die Kulturbrühe wurde steril entnommen und bei 8000 rpm mit 500 ml in 1000 ml-Zentrifugenbechern abzentrifugiert. Der Überstand wurde verworfen, die Pellets wurden in Falcon-Röhrchen mit je 10 g aliquotiert. Die Pellets wurden bei -80 °C für spätere Verwendung eingefroren.

### b) Oxidation von n-Butan zu 1-Butanol und Buttersäure

Es wurden 10 g der wie in a) beschrieben eingefrorenen Biomasse in 50 ml 70 mM Ammoniumphosphatpuffer pH 7 resuspendiert (Zusammensetzung: 8 g/l (NH₄)H₂PO₄, 0,5 g/l NaCl, 0,49g/l MgSO₄ x 7H₂O, 1 ml Spurenelementelösung US3 und 50 µg/l Kanamycin. Die pH-Einstellung erfolgte hier mit 5 % NH₄OH.

Es wurden 130 ml 70 mM Ammoniumphosphatpuffer pH 7 (Zusammensetzung: 8 g/l (NH₄)H₂PO₄, 0,5 g/l NaCl, 0,49 g/l MgSO₄ x 7H₂O, 1 ml Spurenelementelösung US3 und 50 µg/l Kanamycin. pH-Einstellung auf 7,0 mit 5 % Ammoniaklösung.) mit ca. 3 Tropfen autoklaviertem Antischaum (Delamex) in einem 300 ml-Fermenter vorgelegt. Der Fermenter wurde mit einem Gasgemisch bestehend aus 25 % n-Butan und 75 % synthetischer Luft über einen Metallsinterperlator mit einer Porengröße von 0,2 µm mit einer Flussrate von 9 IN/h begast. Der Fermenter wurde in einem Wasserbad auf 30 °C temperiert und mit Hilfe eines Magnetrührers mit 900 rpm gerührt. Der pH-Wert wurde mit 2,5%iger Ammoniaklösung auf 7,0 geregelt. Glucoselösung wurde kontinuierlich zugefüttert (Glucose-Feedrate Rate von 0,9 g/lh). Die Abluft wurde durch eine eisgekühlte Waschflasche, die mit 150 ml Wasser gefüllt ist, abgeleitet.

Von der wie in a) beschriebenen gefrorenen Biomasse wurden 10 g in 50 ml Ammoniumphosphatpuffer resuspendiert und aufgetaut. Mit der Suspension wurde der Fermenter angeimpft.

Die Biomassekonzentration lag bei einer optischen Dichte (600 nm) von 25. Zu verschiedenen Zeitpunkten wurden aus dem Fermenter jeweils 5 ml Probe gezogen. Die Proben wurden für 10 Minuten bei 10000 g und Raumtemperatur zentrifugiert und der Überstand mit einem 0,2 µm-Spritzenvorsatzfilter filtriert.

Die Analyse von Buttersäure und 1-Butanol erfolgte chromatographisch mit HPLC-RID an einer Agilent Technologies 1200-Anlage. Es wurde eine Aminex HPX-87H-Säule (300 mm x 7,8 mm) verwendet. Die Anlage wurde mit 10 mM H₂SO₄ als Laufmittel bei einer Flussrate von 0,6 ml/min und einer Säulentemperatur von 40 °C betrieben. Standards aller zu analysierender Stoffe wurden in Reinstwasser vorbereitet und unter identischen Bedingungen gemessen. Die Auswertung erfolgte über Vergleich der Retentionszeiten.

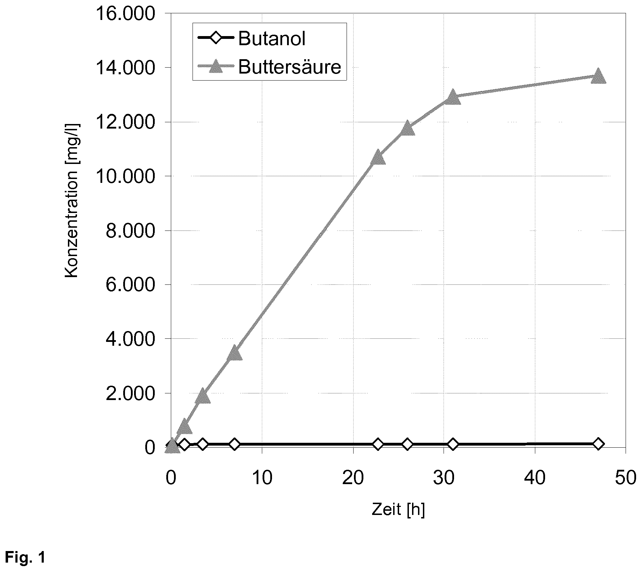

Nach 48 h betrug die Buttersäure-Konzentration ca. 13,5 g/l und die 1-Butanol-Konzentration 0,125 g/l (siehe **Fig. 1**).

### Beispiel 1c: Oxidation von n-Butan durch E. coli W3110 pBT10 mit der Monooxygenase CYP153 aus Alcanivorax borkumensis.

Drei 100-ml Schikanekolben mit 25 ml LB-Medium mit Kanamycin (Zusammensetzung: 5g/l Hefeextrakt, 10 g/l Pepton, 0,5 g/l NaCl, 50 mg/l Kanamycin-Sulfat) werden mit jeweils 100µl einer Glycerin-Kryokultur von E.coli W3110 pCOM10[Ab_Fd / CYP153-2 / FdOR / alkL] mit der Monooxygenase CYP153 aus *Alcanivorax borkumensis* beimpft und für 20 h bei 37 °C und 200 rpm inkubiert. Der eingesetzte Stamm wird in Beispiel 4 in Verbindung mit Beispiel 1 der PCT/EP2013/054928 (WO2013/135650) ausführlich beschrieben. Anschließend wird je 25 ml der Kulturbrühe in 75 ml modifiziertes M9-Medium (Zusammensetzung: 15 g/l Glucose, 6,8 g/l Na₂PO₄, 3 g/l KH₂PO₄, 0,5 g/l NaCl, 2 g/l NH₄Cl, 15 g/l Hefeextrakt, 0,49 g/l MgSO₄*7H₂O, 50 mg/l Kanamycin-Sulfat, 15 ml/l Spurenelementlösung US3. Zusammensetzung der Spurenelementlösung: 36,5 g/l 37%ige Salzsäure, 1,91 g/l MnCl₂*4H₂O, 1,87 g/l ZnSO₄*7H₂O, 0,84 g/l Na-EDTA*2H₂O, 0,3 g/l H₃BO₃, 0,25 g/l Na₂MoO₄*2H₂O, 4,7 g/l CaCl₂*2H₂O, 17,3 g/l FeSO₄*7H₂O, 0,15 g/l CuCl₂*2H₂O) in 1000 ml-Schikanekolben überimpft. Die Kolben wird für 2,5 h bei 37 °C und 200 rpm inkubiert. Danach wird die Temperatur auf 25 °C reduziert. Nach 0,5 Stunden bei 25 °C erfolgt die Induktion mit 0,4 mM Dicyclopropylketon. Die Kulturen werden für weitere 16 h bei 25 °C und 200 rpm inkubiert.

Die Kulturen werden vereinigt, in 50 ml Falcon Tubes überführt und bei 5500 g bei 25 °C für 10 Minuten zentrifugiert. Der Überstand wird verworfen, die Pellets aus 300ml Kultur werden in 50 ml Konversionspuffer pH 7 resuspendiert. (Zusammensetzung: 8 g/l (NH₄)H₂PO₄, 0,5 g/l NaCl, 0,49 g/l MgSO₄*7H₂O, 50 mg/l Kanamycin-Sulfat, 15 ml/l Spurenelementlösung US3; pH-Wert mit 25%iger Ammoniaklösung eingestellt auf 7,0).

Die Transformation, Probenahme und Analytik werden mit *E.coli* W3110 pCOM10[Ab_Fd / CYP153-2 / FdOR / alkL] analog zu 1.1 durchgeführt.

Die Buttersäure und das Butanol aus 1-b) und 1-c) werden destillativ von der wässrigen Phase getrennt und in Beispiel 1d weiter eingesetzt.

### Beispiel 1d: Azeotrope Veresterung von Buttersäure und Butanol

88 g Buttersäure und 81,4 g 1-Butanol aus den Beispielen 1b und 1c und im Falle des Butanols gegebenenfalls aus Beispiel 3 sowie 5 g Paratoluensulfonsäure werden gemischt und zum Sieden erhitzt. Die Reaktion wird unter Rückfluss mittels azeotroper Destillation mit Wasserabscheider durchgeführt, bis die berechnete Menge Wasser (18 mL) für den quantitativen Umsatz abgeschieden wurde. Der gebildete Ester (129,6 g Butylbutyrat) wird destillativ von überschüssigem 1-Butanol und dem Katalysator getrennt und in Beispiel 2 zur Oxidation mittels Ganzzellkatalysator überführt.

### Beispiel 2: Oxidation von Butylbutyrat

Je ein 100-ml Schikanekolben mit 25 ml LB-Medium mit Kanamycin (Zusammensetzung: 5 g/l Hefeextrakt, 10 g/l Pepton, 0,5 g/l NaCl, 50 mg/l Kanamycin-Sulfat) wurden mit jeweils 100 µl einer Glycerin-Kryokultur von *E.coli* W3110 pCOM10[Ab_Fd / CYP153-2 / FdOR / alkL] bzw. von *E.coli* W3110 pBT10 beimpft und für 20 h bei 37 °C und 200 rpm inkubiert.

Anschließend wurden die kompletten Vorkulturen jeweils in 75 ml modifiziertes M9-Medium (Zusammensetzung: 15 g/l Glucose, 6,8 g/l Na₂PO₄, 3 g/l KH₂PO₄, 0,5 g/l NaCl, 2 g/l NH₄Cl, 15 g/l Hefeextrakt, 0,49 g/l MgSO₄*7H₂O, 50 mg/l Kanamycin-Sulfat, 15 ml/l Spurenelementlösung US3. Zusammensetzung der Spurenelementlösung: 36,5 g/l 37%ige Salzsäure, 1,91 g/l MnCl₂*4H₂O, 1,87 g/l ZnSO₄*7H₂O, 0,84 g/l Na-EDTA*2H₂O, 0,3 g/l H₃BO₃, 0,25 g/l Na₂MoO₄*2H₂O, 4,7 g/l CaCl₂*2H₂O, 17,3 g/l FeSO₄*7H₂O, 0,15 g/l CuCl₂*2H₂O) in 1000 ml-Schikanekolben überimpft. Die Kolben wurden für 2,5 h bei 37 °C und 200 rpm inkubiert. Danach wurde die Temperatur auf 25 °C reduziert. Nach 0,5 Stunden erfolgte die Induktion mit 0,4 mM Dicyclopropylketon. Die Kultur wurde für weitere 16 h bei 25 °C und 200 rpm inkubiert.

Die Kulturen wurden in 50ml Falcon Tubes überführt und bei 5500g bei 25 °C für 10 Minuten zentrifugiert. Der Überstand wurde verworfen, die Pellets wurden je Stamm in 50 ml Konversionspuffer pH 7 resuspendiert. (Zusammensetzung: 8 g/l (NH₄)H₂PO₄, 0,5 g/l NaCl, 0,49 g/l MgSO₄*7H₂O, 50 mg/l Kanamycin-Sulfat, 15 ml/l Spurenelementlösung US3; pH-Wert mit 25%iger Ammoniaklösung eingestellt auf 7,0) und in 500 ml-Schikanekolben bei 30 °C und 180 rpm inkubiert.

Um die Reaktion zu starten wurden je 2 g/l Butylbutyrat sowie 5 g/l Glucose zugegeben. Als Negativkontrolle wurde ein Kolben mit Puffer, Butylbutyrat und Glucose ohne Zellen inkubiert.

Die Biomassekonzentration lag bei einer optischen Dichte (600 nm) von 2,5. Nach 10 min und 7 h wurde jeweils 2 ml Probe gezogen. Die Proben wurden für 10 Minuten bei 10000 g und Raumtemperatur zentrifugiert und der Überstand mit einem 0,2 µm-Spritzenvorsatzfilter filtriert.

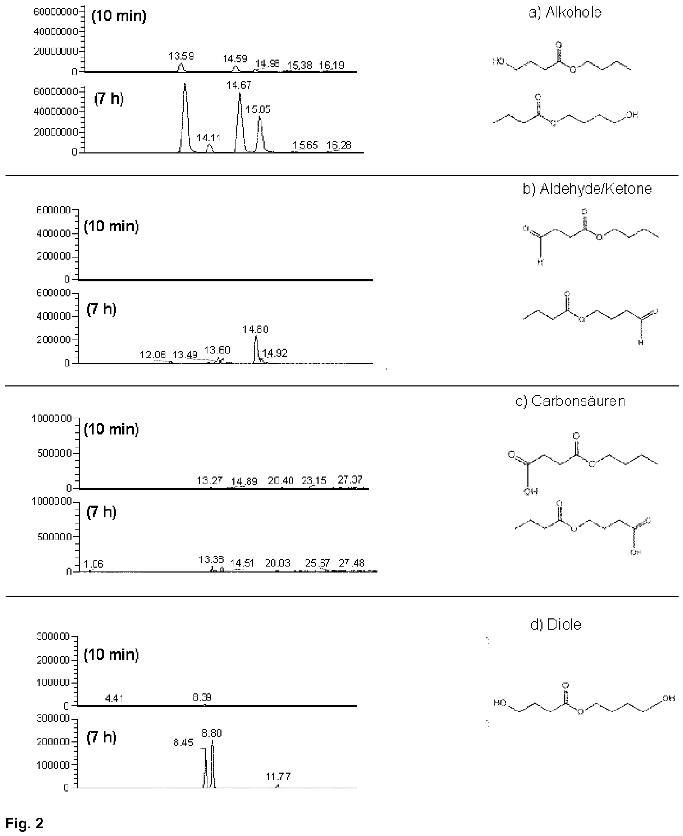

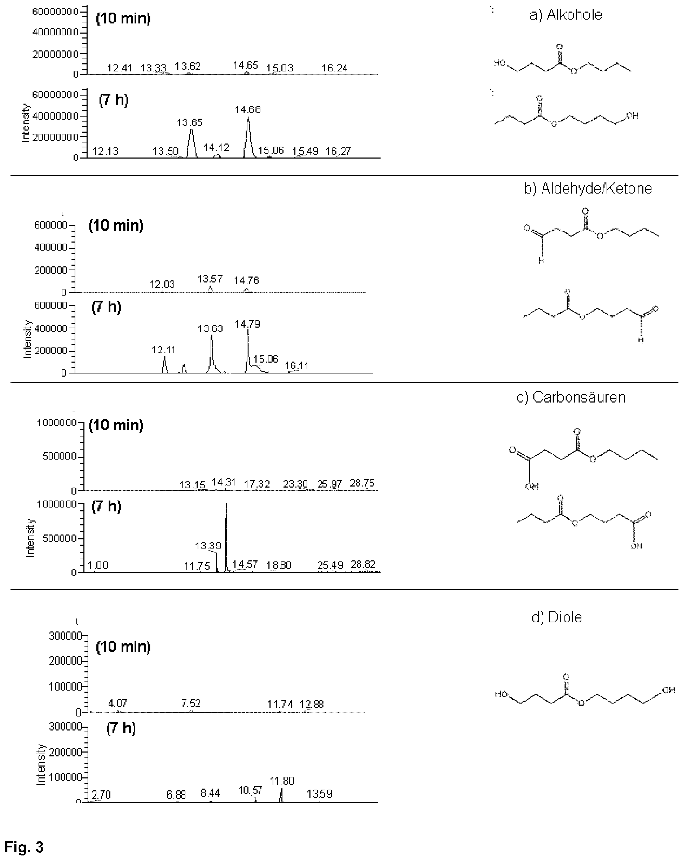

Ein Screening nach Oxidationsprodukten von Butylbutyrat erfolgte mit HPLC-ESI-MS (Thermo Fisher Scientific). Anhand ihrer akkuraten Massen und den daraus abgeleiteten Summenformeln konnten Butylbutyrat und die daraus entstehenden Oxidationsprodukte identifiziert werden. In beiden Versuchsansätzen konnte u.a. der einfach und zweifach terminal hydroxylierte Ester nachgewiesen werden (siehe **Figs. 2** und **3**).

### Beispiel 3: Reduktive Hydrieren des oxidierten Butylbutyrats unter Entstehung von 1,4-Butandiol:

Es werden 740mg Lithiumaluminiumhydrid in 50 mL trockenem Diethylether vorgelegt und 10 g de oxidierten Ester aus Beispiel 2 langsam bei 0 °C zugetropft. Nach vollständiger Zugabe wird die Mischung zum Sieden erhitzt und unter Rückfluss bis zum vollständigen Umsatz gerührt. Die entstehenden Alkohole werden destillativ getrennt. Man erhält 7.41 g 1,4-Butandiol und 1,77 g 1-Butanol. Das Butanol wird zu Beispiel 1d zurückgeführt und dort verestert.

## Patentansprüche

1. Verfahren zur Herstellung von α,ω-Alkandiol umfassend die Schritte
a) Umsetzen einer Alkansäure mit einem Alkanol zu einem Ester,
b) Oxidation wenigstens eines terminalen Kohlenstoffatoms des Esters durch Kontaktieren mit einem Ganzzellkatalysator, der eine Alkanhydroxylase exprimiert, in wässriger Lösung und in Gegenwart von molekularem Sauerstoff, zu einem oxidierten Ester,
c) Hydrieren des oxidierten Esters unter Entstehung eines α,ω-Alkandiols und Alkanols, und
d) Destillatives Abtrennen des Alkanols unter Entstehung einer mit Hinblick auf das Alkanol abgereicherten Reaktionsmischung, und
e) Rückführen des Alkanols in Schritt a).

2. Verfahren nach Anspruch 1, wobei es sich bei der Alkansäure in Schritt a) um eine Alkansäure der Formel H₃C - (CH₂)ₙ - COOH handelt, und wobei n > 0, bevorzugt 2 bis 16 ist.

3. Verfahren nach Anspruch 1, wobei es sich bei der Alkansäure um Buttersäure handelt.

4. Verfahren nach Anspruch 1, wobei es sich bei der Alkansäure in Schritt a) um ein carboxyliertes Cycloalkan der Formel CₙH₂ₙ₋₁COOH handelt, wobei n > 4, bevorzugt 5 bis 8.

5. Verfahren nach einem der Ansprüche 1 bis 4, wobei es sich bei dem Alkanol um ein Alkanol der Formel CₙOH₂ₙ₊₂ handelt und n > 0 ist.

6. Verfahren nach einem der Ansprüche 1 bis 5, wobei die Alkansäure und das Alkanol in Schritt a) das gleiche Kohlenstoffgerüst aufweisen.

7. Verfahren nach einem der Ansprüche 1 bis 6, wobei der Ganzzellkatalysator in Schritt a) eine Alkanhydroxylase exprimiert, die aus der Gruppe umfassend AlkB aus *Pseudomonas putida* und Varianten davon und CYP153 aus *Alcanivorans borkumensis* SK2 und Varianten davon ausgewählt ist, wobei eine Variante der jeweiligen vorgenannten Aminosäuresequenz eine Sequenz umfasst, welche mit Hinblick auf die entsprechende ursprüngliche Wildtypaminosäuresequenz eine Identität von 70% oder mehr Prozent aufweist.

8. Verfahren nach einem der Ansprüche 1 bis 7, wobei Schritt a) mittels säurekatalysierter Esterbildung durchgeführt wird, bevorzugt in Anwesenheit eines organischen Lösungsmittels.

9. Verfahren nach einem der Ansprüche 1 bis 8, wobei Schritt a) mittels Kontaktieren mit einer Esterase, Protease oder Lipase, bevorzugt bereitgestellt in Form eines Ganzzellkatalysators exprimierend die Esterase oder Lipase, in wässriger Lösung durchgeführt wird.

10. Verfahren nach einem der Ansprüche 1 bis 9, wobei die Alkansäure in Schritt a) durch Oxidation eines Alkans zur Alkansäure bereitgestellt wird.

## Claims

1. Process for preparing α-ω-alkanediol comprising the steps of:
a) reacting an alkanoic acid with an alkanol to give an ester,
b) oxidizing at least one terminal carbon atom of the ester by contacting with a whole-cell catalyst, which expresses an alkane hydroxylase, in aqueous solution and in the presence of molecular oxygen, to give an oxidized ester,
c) hydrogenating the oxidized ester to form an α, ω-alkanediol and alkanol, and
d) removing the alkanol by distillation, forming a reaction mixture depleted with respect to the alkanol, and
e) recycling the alkanol in step a).

2. Process according to Claim 1, wherein the alkanoic acid in step a) is an alkanoic acid of the formula H₃C - (CH₂) ₙ - COOH, and where n > 0, preferably 2 to 16.

3. Process according to Claim 1, wherein the alkanoic acid is butanoic acid.

4. Process according to Claim 1, wherein the alkanoic acid in step a) is a carboxylated cycloalkane of the formula CₙH₂ₙ₋₁COOH, where n > 4, preferably 5 to 8.

5. Process according to any of Claims 1 to 4, wherein the alkanol is an alkanol of the formula CₙOH₂ₙ₊₂ and n > 0.

6. Process according to any of Claims 1 to 5, wherein the alkanoic acid and the alkanol in step a) have the same carbon skeleton.

7. Process according to any of Claims 1 to 6, wherein the whole-cell catalyst in step a) expresses an alkane hydroxylase selected from the group comprising AlkB from *Pseudomonas putida* and variants thereof and CYP153 from *Alcanivorans borkumensis* SK2 and variants thereof, wherein a variant of the respective abovementioned amino acid sequence comprises a sequence which, with respect to the corresponding original wild-type amino acid sequence, has an identity of 70% or greater.

8. Process according to any of Claims 1 to 7, wherein step a) is carried out by means of acid-catalysed ester formation, preferably in the presence of an organic solvent.

9. Process according to any of Claims 1 to 8, wherein step a) is carried out in aqueous solution by means of contacting with an esterase, protease or lipase, preferably provided in the form of a whole-cell catalyst expressing the esterase or lipase.

10. Process according to any of Claims 1 to 9, wherein the alkanoic acid in step a) is provided by oxidizing an alkane to the alkanoic acid.

## Revendications

1. Procédé de fabrication d'un α, ω-alcanediol comprenant les étapes suivantes :
a) la mise en réaction d'un acide alcanoïque avec un alcanol pour former un ester,
b) l'oxydation d'au moins un atome de carbone terminal de l'ester par mise en contact avec un catalyseur à cellules entières, qui exprime une alcane hydroxylase, dans une solution aqueuse et en présence d'oxygène moléculaire, pour former un ester oxydé,
c) l'hydrogénation de l'ester oxydé avec formation d'un α, ω-alcanediol et d'un alcanol, et
d) la séparation par distillation de l'alcanol avec formation d'un mélange réactionnel appauvri au regard de l'alcanol, et
e) le recyclage de l'alcanol dans l'étape a).

2. Procédé selon la revendication 1, dans lequel l'acide alcanoïque à l'étape a) est un acide alcanoïque de formule H₃C-(CH₂)ₙ-COOH, et n > 0, de préférence 2 à 16.

3. Procédé selon la revendication 1, dans lequel l'acide alcanoïque est l'acide butyrique.

4. Procédé selon la revendication 1, dans lequel l'acide alcanoïque à l'étape a) est un cycloalcane carboxylé de formule CₙH₂ₙ₋₁COOH, avec n > 4, de préférence 5 à 8.

5. Procédé selon l'une quelconque des revendications 1 à 4, dans lequel l'alcanol est un alcanol de formule CₙOH₂ₙ₊₂ et n > 0.

6. Procédé selon l'une quelconque des revendications 1 à 5, dans lequel l'acide alcanoïque et l'alcanol à l'étape a) présentent le même squelette carboné.

7. Procédé selon l'une quelconque des revendications 1 à 6, dans lequel le catalyseur à cellules entières à l'étape a) exprime une alcane hydroxylase qui est choisie dans le groupe comprenant AlkB de *Pseudomonas putida* et ses variantes et CYP153 d'*Alcanivorans borkumensis* SK2 et ses variantes, une variante de la séquence d'acides aminés susmentionnée respective comprenant une séquence qui présente une identité de 70 % ou plus avec la séquence d'acides aminés de type sauvage initiale correspondante.

8. Procédé selon l'une quelconque des revendications 1 à 7, dans lequel l'étape a) est réalisée par formation d'ester sous catalyse acide, de préférence en présence d'un solvant organique.

9. Procédé selon l'une quelconque des revendications 1 à 8, dans lequel l'étape a) est réalisée par mise en contact avec une estérase, une protéase ou une lipase, de préférence préparée sous la forme d'un catalyseur à cellules entières exprimant l'estérase ou la lipase, dans une solution aqueuse.

10. Procédé selon l'une quelconque des revendications 1 à 9, dans lequel l'acide alcanoïque à l'étape a) est préparé par oxydation d'un alcane en l'acide alcanoïque.
